# EUROPEAN PATENT APPLICATION

(11) **EP 2 499 973 A1**
(43) Date of publication of application: **19.09.2012**
(21) Application number: 10829915.7
(22) Date of filing: 09.11.2010
(51) Int. Cl.: A61B 8/00

(54) **ULTRASONIC DIAGNOSIS DEVICE**

(30) Priority: 10.11.2009 JP 2009257105
(71) Applicant: Hitachi Medical Corporation, Chiyoda-ku Tokyo 101-0021 (JP)
(72) Inventor: OSHIKI, Mitsuhiro, Tokyo 101-0021 (JP); KISHI, Shinichiro, Tokyo 101-0021 (JP); SHINOMARU, Hideki, Tokyo 101-0021 (JP)
(74) Representative: Strehl Schübel-Hopf & Partner
(86) International application number: PCT/JP2010/069882
(87) International publication number: WO 2011/058956

(57) **Abstract**

An ultrasonic diagnostic apparatus of the present invention includes a signal transmission section, which supplies a driving pulse to a transducer in order to transmit an ultrasonic transmission signal to an object and transmits to the object a non-inverted component and an inverted component of an ultrasonic waveform, and a signal processing section, which removes a fundamental wave component by adding received signals from the object. The signal transmission section includes a switching section which changes an output period within a half period of the transmission signal according to at least one of a load connected to the signal transmission section and amplitude, a frequency, and a wave number output from the transmission circuit.

## Description

### Technical Field

The present invention relates to an ultrasonic diagnostic apparatus having a function of transmitting a transmission signal with a fundamental frequency to an object and imaging the harmonics of a received signal from the object (harmonic imaging) and in particular, to a technique for suppressing the noise of harmonic components caused by the asymmetry of a non-inverted component and an inverted component of the transmission signal.

### Background Art

An ultrasonic diagnostic apparatus applies a voltage to a transducer, transmits to an object an ultrasonic wave generated by the voltage application to the transducer, and extracts a reflected signal from the object to acquire the tomographic information or the like regarding the inside of the object from the extracted reflected signal.

In the ultrasonic diagnostic apparatus, there is a harmonic imaging technique for receiving a harmonic 2f0 of a frequency (fundamental frequency f0) of a transmission signal. Tissue harmonic imaging, contrast imaging, and pulse inversion imaging are known as the harmonic imaging techniques.

In harmonic imaging, it has been demanded to reduce the generation of the noise of harmonics of a signal transmission circuit in order to suppress the degradation of image quality of an image obtained using harmonics generated from the inside of the living body or from the contrast agent. A method of the solving means is disclosed in PTL 1.

PTL 1 discloses that an output signal with a waveform, which has less harmonic components than a waveform of an output signal from a signal transmission circuit in the conventional example, can be generated by changing a voltage, which is applied between one end and a center tap that bisects the number of windings of the primary winding of a transformer, and a voltage, which is applied between the center tap and the other end, and accordingly the degradation of image quality can be suppressed in tissue harmonic imaging or contrast imaging.

### Citation List

### Patent Literature

[PTL 1] JP-A-2002-315748

### Outline of Invention

### Problem to be Solved by the Invention

In PTL 1, however, there is no consideration regarding the configuration for reducing the noise of harmonics generated when the signal level of an input signal of a signal transmission section changes.

It is an object of the present invention to provide an ultrasonic diagnostic apparatus capable of reducing the noise of harmonics which are generated when the signal level of an input signal to a signal transmission section changes.

### Means for Solving the Problems

In the present invention, in order to achieve the above-described object, a "switching section" is added to a signal transmission circuit (referred to as a "signal transmission section") of an ultrasonic diagnostic apparatus.

Specifically, the signal transmission section includes a switching section which changes an output period within a half period of the transmission signal according to at least one of a load connected to the signal transmission section and amplitude, a frequency, and a wave number output from the signal transmission circuit.

### Advantage of the Invention

According to the present invention, it is possible to reduce the noise of harmonics which is generated when the signal level of an input signal to a signal transmission section changes.

### Brief Description of Drawings

[Fig. 1] Fig. 1 is a block diagram showing the entire configuration of an ultrasonic diagnostic apparatus to which the present invention is applied.
[Fig. 2] Fig. 2 is an explanatory view of a signal transmission circuit for an ultrasonic diagnostic apparatus.
[Fig. 3] Fig. 3 is an explanatory view of a signal transmission circuit for an ultrasonic diagnostic apparatus.
[Fig. 4] Fig. 4 is an explanatory view of a signal transmission circuit for an ultrasonic diagnostic apparatus.
[Fig. 5] Fig. 5 is an explanatory view of a signal transmission circuit for an ultrasonic diagnostic apparatus.
[Fig. 6] Fig. 6 is a view showing an example of the hydrophone waveform of a transmission output signal.
[Fig. 7] Fig. 7 is a view showing an example of the hydrophone waveform of a transmission output signal.
[Fig. 8] Fig. 8 is a view showing the frequency response corresponding to the transmission output signal in Fig. 6.
[Fig. 9] Fig. 9 is a view showing the frequency response corresponding to the transmission output signal in Fig. 7.
[Fig. 10] Fig. 10 is a view showing an example of a signal transmission circuit for an ultrasonic diagnostic apparatus.
[Fig. 11] Fig. 11 is a view showing an example of a transmission output waveform when a probe is connected.
[Fig. 12] Fig. 12 is a view showing an ideal transmission output waveform.
[Fig. 13] Fig. 13 is a view showing an example of a transmission signal generator for changing the duty ratio.
[Fig. 14] Fig. 14 is a view showing another example of a signal transmission circuit for an ultrasonic diagnostic apparatus.
[Fig. 15] Fig. 15 is a view showing a timing chart of a transmission signal for an ultrasonic diagnostic apparatus.
[Fig. 16] Fig. 16 is a view showing the relationship of a transmission signal for an ultrasonic diagnostic apparatus, a harmonic generation rate, and an S/N ratio of a harmonic diagnostic image.

### Mode for Carrying Out Invention

Hereinafter, embodiments of this invention will be described with reference to the drawings.

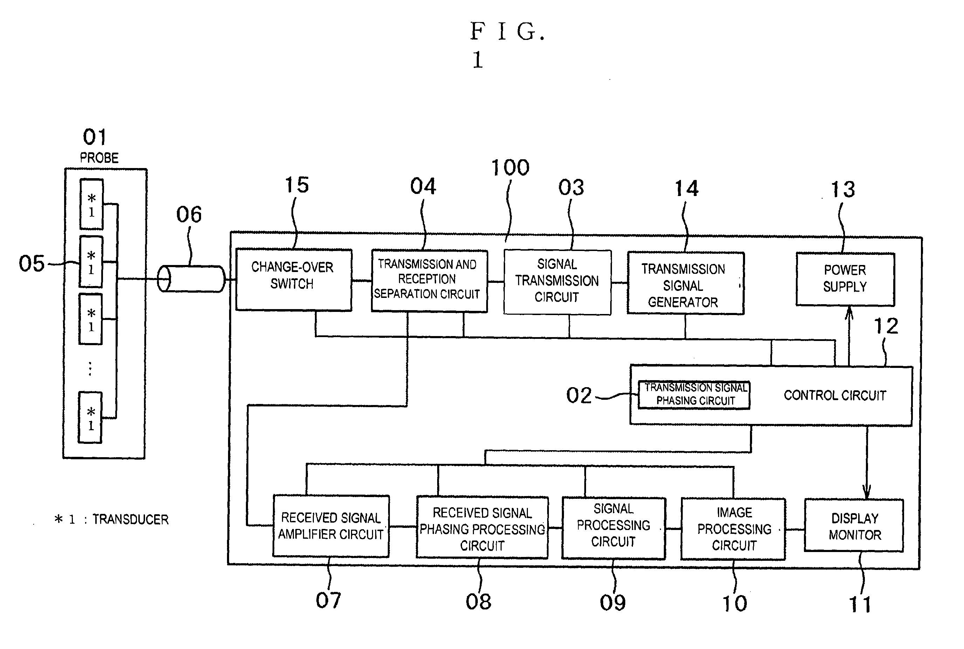

Fig. 1 shows an example of an ultrasonic diagnostic apparatus to which the present invention is applied.

The ultrasonic diagnostic apparatus is configured to include a probe 01 in which a transducer 05 is provided, a transmission signal phasing circuit 02, a signal transmission circuit 03, a transmission and reception separation circuit 04, a cable 06, a received signal amplifier circuit 07, a received signal phasing processing circuit 08, a signal processing circuit 09, an image processing circuit 10, and a display monitor 11.

The transducer 05 is formed to have a function of converting a transmission signal of continuous waves or a pulse wave input from the signal transmission circuit 03 into an ultrasonic wave and transmitting it to the object and a function of receiving an ultrasonic wave reflected from the inside of the object, converting the ultrasonic wave into an electric signal and outputting the electric signal.

The transmission signal phasing circuit 02 is for adjusting the timing of voltage application to each transducer 05 to be driven when forming an ultrasonic beam transmitted to the object. The transmission signal phasing circuit 02 controls the timing such that the larger the distance between the transducer to be driven and the ultrasonic beam focusing position, the earlier the voltage application time.

The signal transmission circuit 03 is for amplifying a transmission signal waveform, which is formed by a transmission signal generator 14, up to the sufficient strength to generate an ultrasonic signal by driving the transducer 05.

The transmission signal generator 14 generates a waveform of a transmission signal. The transmission signal generator 14 includes a memory to store one or plural transmission waveforms, for example.

In addition, a console (not shown) used when an operator operates an ultrasonic diagnostic apparatus is provided in an ultrasonic diagnostic apparatus body 100, and a waveform can be selected by a control circuit 12 which receives an input from the console. Alternatively, a waveform may be transmitted from the ultrasonic diagnostic apparatus body 100 through the cable 06 whenever a transmission signal is generated and be stored in the memory.

The transmission and reception separation circuit 04 changes the signal path on the circuit when guiding to the received signal amplifier circuit 07 a signal transmitted by the signal transmission circuit 03 and a received signal that is a reflected signal of an ultrasonic signal, which is transmitted by driving of the transducer 05 using the transmitted signal, from the object.

Due to the transmission and reception separation circuit 04, the signal from the signal transmission circuit 03 can be transmitted to the transducer 05 without being applied to the received signal amplifier circuit 07, and the signal from the object can be guided to the received signal amplifier circuit 07 through the transducer 05 without influence of the signal transmission circuit 03.

The received signal amplifier circuit 07 amplifies an ultrasonic signal acquired from the object, and also has a function of changing the gain each time.

The received signal phasing processing circuit 08 has a function of forming an ultrasonic beam of the received signal, similar to the transmission signal phasing circuit 02. The received signal phasing processing circuit 08 is for adjusting the addition timing of signals from the transducers 05 of all channels, in which the signal from the object is acquired, for each transducer 05 when forming an ultrasonic beam of the received signal. The timing of addition of the received signal acquired by the transducer 05, which is far from the focus position, is adjusted by increasing the delay time, which is given to the output signal that is output from the transducer 05, in inverse proportion to the distance between the transducer 05 and the focus position.

The signal processing circuit 09 and the image processing circuit 10 are for performing coordinate transformation processing according to the type of the probe 01 by performing signal processing for converting phase-adjusted and added signals into brightness information by detection processing or the like and performing image signal processing represented by gamma (y) processing or the like. The signal processed by the signal processing circuit 09 and the image processing circuit is displayed as a diagnostic image on the display monitor 11.

In addition, each circuit described above receives a basic clock signal from the control circuit 12, so that timing control or the like of each section is performed. Specifically, switching control of transmission and reception or switching of a diagnostic mode is performed.

In addition, a power supply 13 is controlled by the control circuit 12 so as to output various voltage values or current values. Electric power with various voltage values or current values generated herein is supplied to each circuit section (not shown).

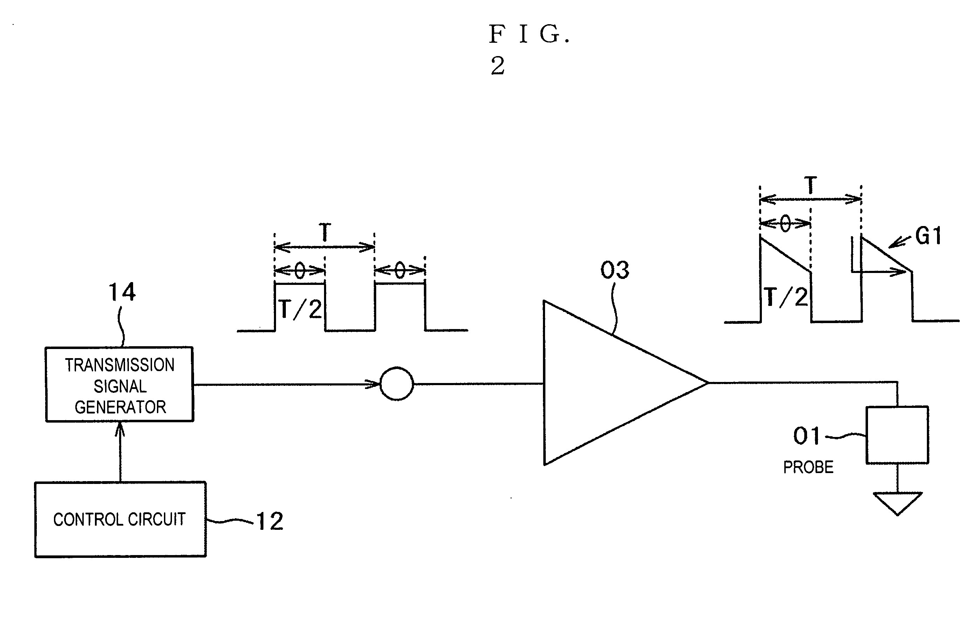

Figs. 2 to 4 show explanatory views of constituent sections regarding the present invention.
Fig. 2 shows a case where a transmission signal with a period T and a duty ratio of 50% is input from the transmission signal generator 14 to the signal transmission circuit 03. Incidentally, the duty ratio is defined as a ratio of an output period of a signal with a value occupied in one period in a periodic input signal. The duty ratio is transmitted from the control circuit 12 to the transmission signal generator 14. A signal amplified by a desired gain has an inclination G1 depending on a time constant determined by the load including the signal transmission circuit 03 and the connected probe 01.

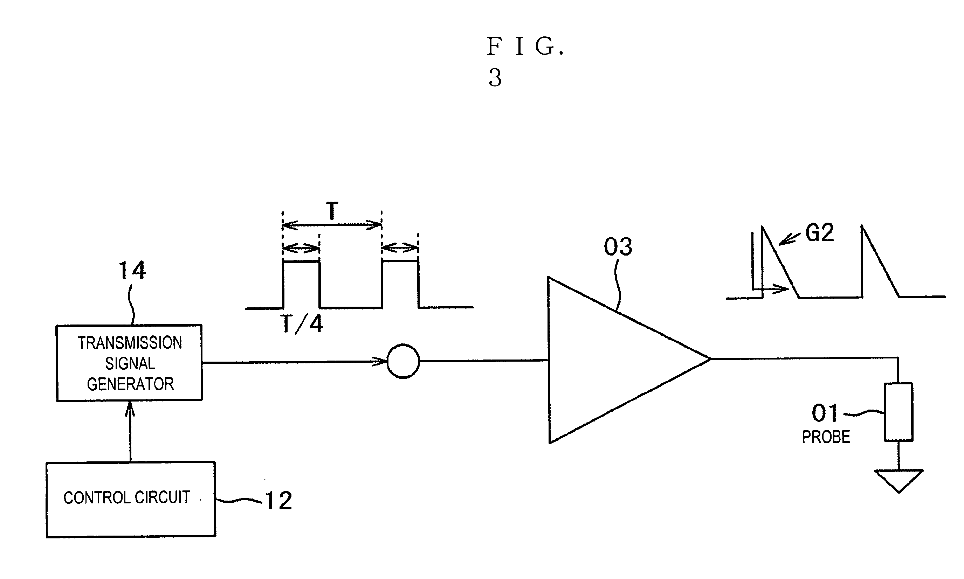

On the other hand, Fig. 3 shows a case where a transmission signal with a period T and a duty ratio of 25% is input from the transmission signal generator 14 to the signal transmission circuit 03. The duty ratio is transmitted from the control circuit 12 to the transmission signal generator 14 as in Fig. 2.

A signal amplified by a desired gain has an inclination G2 by a time constant determined by the load including the signal transmission circuit 03 and the connected probe 01. Since only the probe 01 which is a connected load is different in Figs. 2 and 3, the only other setting which is different is the duty ratio.

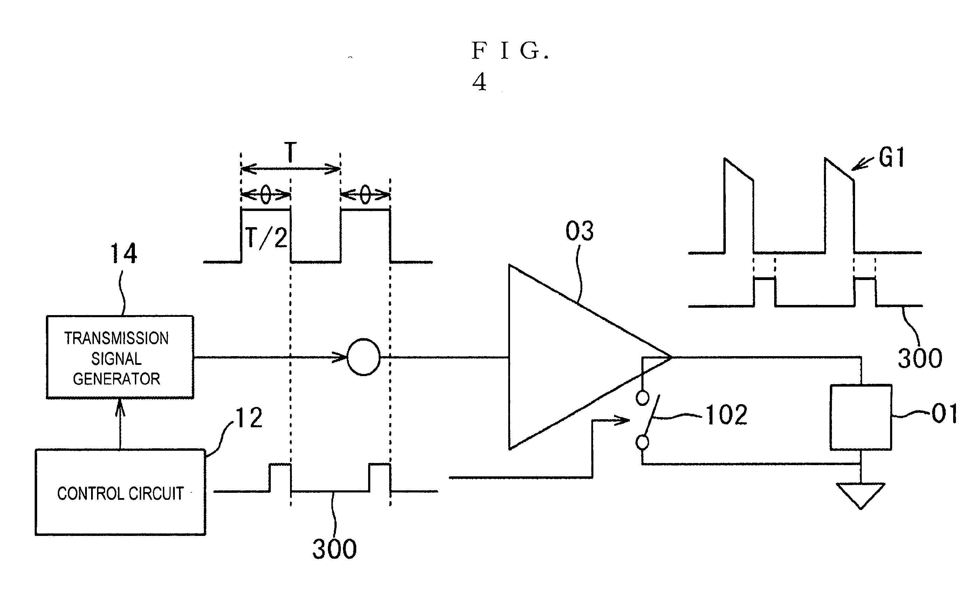

Fig. 4 shows a case where a transmission signal with a period T and a duty ratio of 50% is input from the transmission signal generator 14 to the signal transmission circuit 03 as in Fig. 2. The duty ratio is transmitted from the control circuit 12 to the transmission signal generator 14. A signal amplified by a desired gain has an inclination G1 by a time constant determined by the load including the signal transmission circuit 03 and the connected probe 01.

On the other hand, in Fig. 4, a ground switch 102 is provided in an output section of the signal transmission circuit 03, unlike Fig. 2. This switch 102 is turned on only when the value of a voltage with amplitude equal to or larger than a certain fixed value is input, so that the output section of the signal transmission circuit 03 maintains a ground level only during this period.

Now, it is assumed that a signal 300 is input from the control circuit 12 to the switch 102. When the signal 300 does not have a value, the output of the signal transmission circuit 03 has the same waveform as in Fig. 2, which is determined by an input signal with the period T and the duty ratio of 50%.

On the other hand, in a section in which the signal 300 has a voltage value of amplitude (hereinafter, simply referred to as a "value"), that is, in a section in which the signal 300 overlaps a second half of a section in which an input signal of the signal transmission circuit 03 has a value, the switch 102 is turned on, so that an output signal of the signal transmission circuit 03 flows to the ground through the switch 102. Accordingly, the output signal of the signal transmission circuit 03 has no value.

By controlling the switch 102 as described above, the output signal of the signal transmission circuit 03 can be set to have an arbitrary duty ratio without being limited to the duty ratio of the input signal. In other words, the output signal of the signal transmission circuit 03 can be set to have an arbitrary waveform.

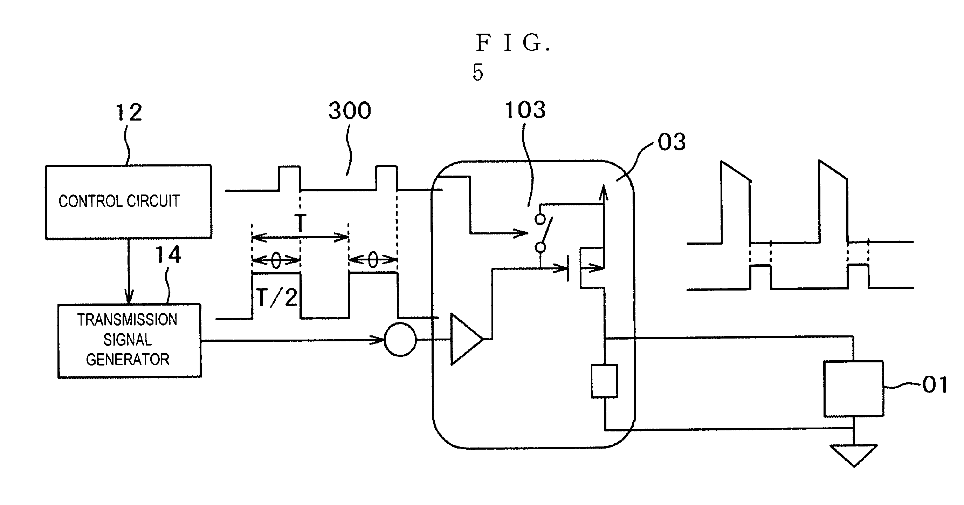

Fig. 5 shows an example of duty ratio control of an output signal in a configuration different from Fig. 4. A transmission signal with a period T and a duty ratio of 50% is input from the transmission signal generator 14 to the signal transmission circuit 03.
The duty ratio is transmitted from the control circuit 12 to the transmission signal generator 14.

In Fig. 5, an output end of the signal transmission circuit 03 is formed by a field effect transistor (FET), and an output signal is formed only for a period for which the FET is turned on by the input signal so that the same output waveform as in Fig. 2 is obtained. On the other hand, the signal 300 output from the control circuit 12 is input to a switch 103 provided between a gate and a source of the FET. Similar to the switch 102, this switch 103 is configured to be turned on when the signal 300 has a value. When the switch 103 is turned on, the gate and the source of the FET is short-circuited regardless of the value of the input signal. Accordingly, the FET does not operate.

Thus, an interval between a non-inverted component and an inverted component of a transmission signal can be controlled by the output signal, without depending on the duty ratio of the input signal.

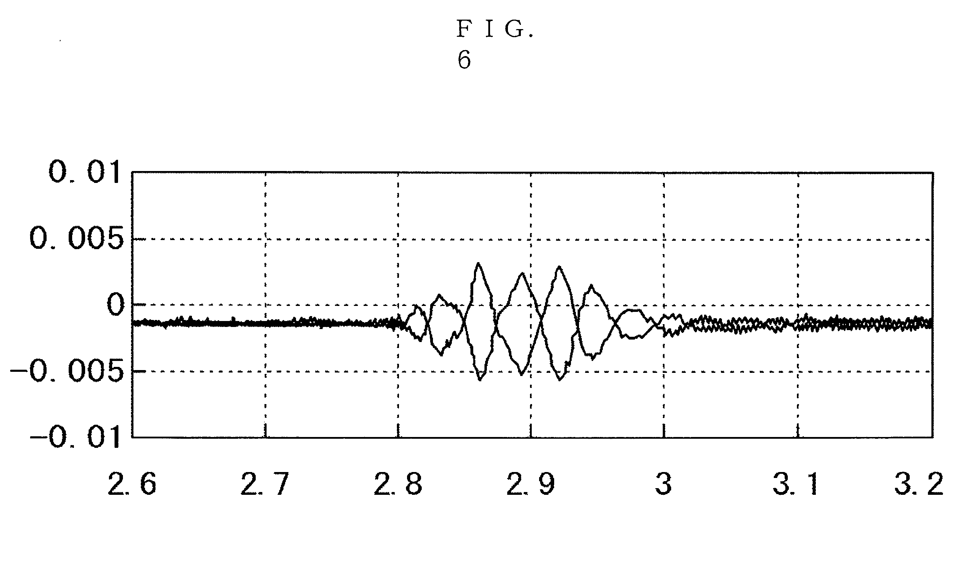

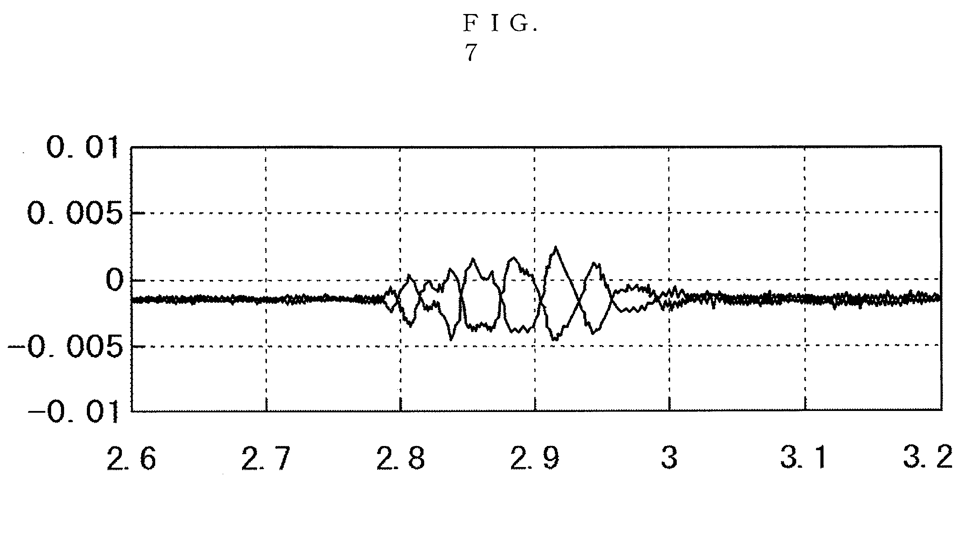

Examples of a hydrophone waveform of a transmission output signal when the duty ratio of an output signal is actually changed by connecting the probe 01 are shown in Figs. 6 and 7. In Figs. 6 and 7, only the duty ratio of the transmission output waveform changes. Fig. 6 shows a case where the duty ratio is set to an appropriate value, and Fig. 7 shows a case where the duty ratio is not set to an appropriate value. Figs. 6 and 7 each show a non-inverted waveform and an inverted waveform, but the symmetry of a non-inverted component and an inverted component shown in Fig. 6 is improved compared with that in Fig. 7 and reduction of harmonic components from the addition result of the components is confirmed.

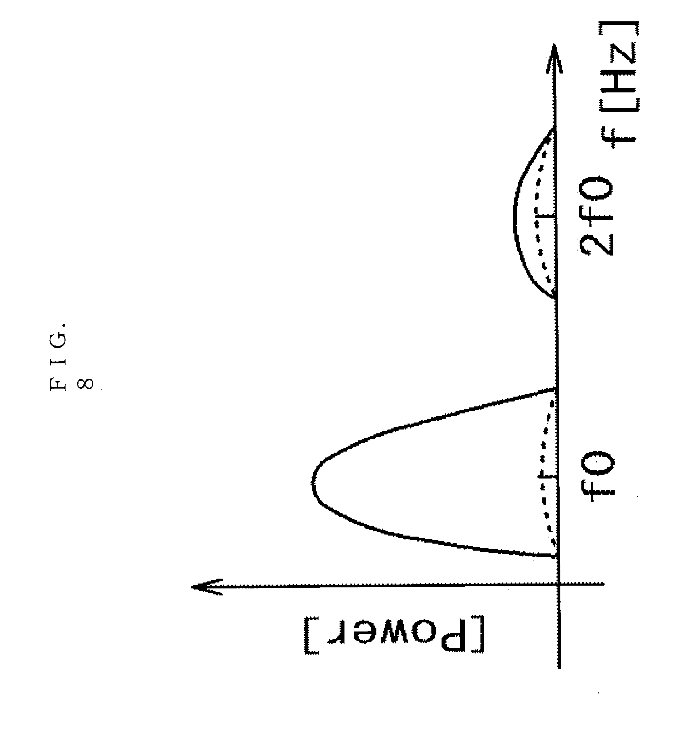

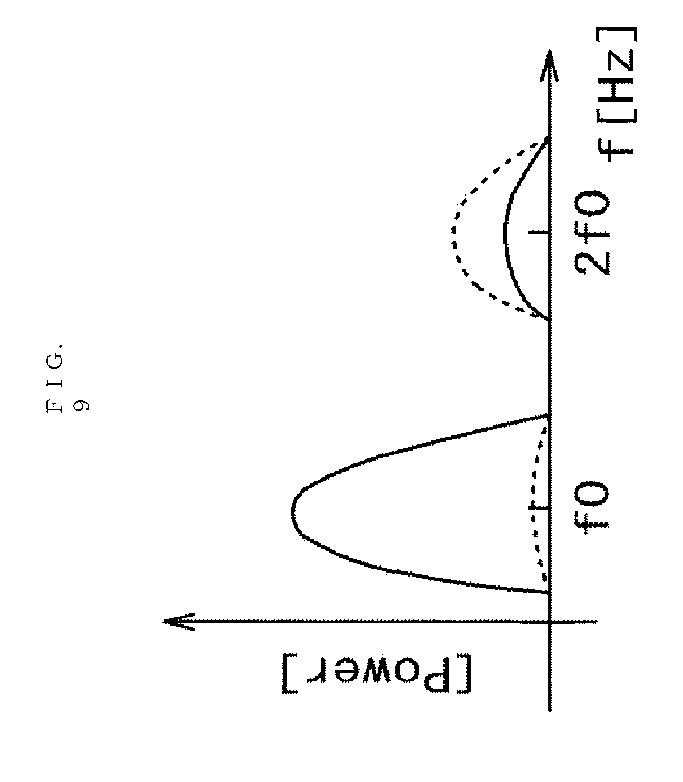

A frequency response corresponding to Fig. 6 is shown in Fig. 8, and a frequency response corresponding to Fig. 7 is shown in Fig. 9.
A solid line shows a frequency distribution of inverted components or non-inverted components, and a dotted line shows a frequency distribution in an addition signal thereof.

In Fig. 7, a non-inverted component and an inverted component are asymmetric, and the frequency distribution of the addition signal includes a harmonic 2f0 component as shown in Fig. 9. As a result, it cannot be determined whether the 2f0 component of the received signal is from a measurement object or a transmission signal since there are many harmonic components of the transmission signal itself, although imaging only the second harmonic component from the measurement object is originally required. For this reason, the S/N ratio of an image is reduced.

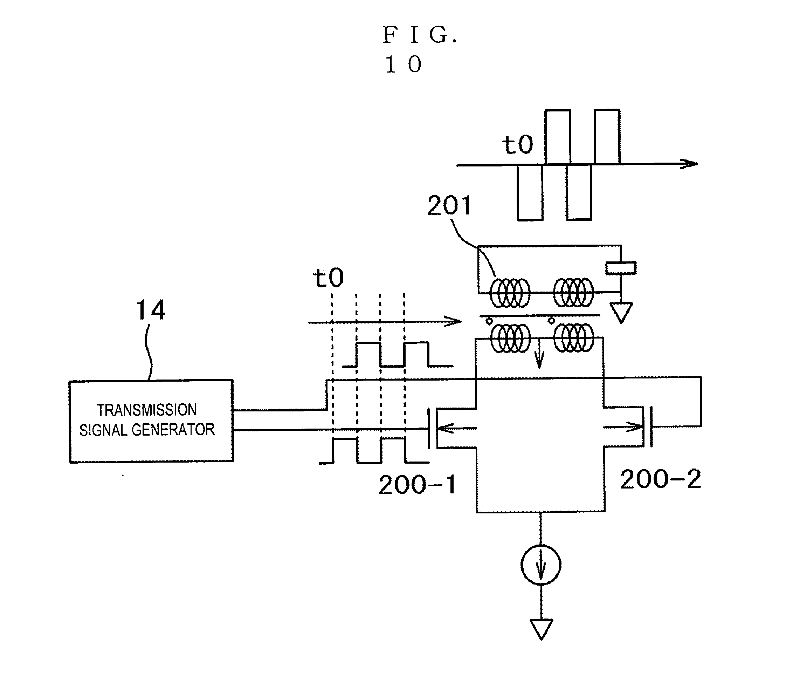

Fig. 10 shows an example where a circuit, which outputs the amplitude of a non-inverted component and the amplitude of an inverted component of a transmission waveform, is formed by components with the same characteristics.

In the configuration shown in Fig. 10, a transformer 201 is adopted. This is a completely symmetrical circuit configuration. FETs with the same polarities and the same package may be used. At the primary side of the transformer to which an FET and the like are connected, a single power supply is prepared in a middle portion of the transformer. At the left and right sides of the circuit, the polarities of the transformer are opposite in order to have both non-inversion and inversion polarities. However, it is possible to use completely the same components in the winding ratio, a material, and the like of the transformer. Input signals to FETs 200-1 and 200-2 are supplied from the transmission signal generator 14. In order to output one polarity of a transmission output signal to each of the FETs 200-1 and 200-2, it is necessary to shift the timing, at which each of the FETs 200-1 and 200-2 is turned on, by the half period of the output signal.

The effect of the above-described reduction of harmonic components from the addition result of non-inverted components and inverted components can be obtained by changing the duty ratio according to at least one of a connected load and the amplitude value, a frequency, and a wave number of an output.

The signal transmission circuit 03 may have a circuit which amplifies an input signal using only one power supply on the non-inversion side or the inversion side, and each output section may be commonly used through an inverting section and a non-inverting section of a transformer, so that the circuit structure is reduced by the common circuit.

Although Figs. 2 and 3 show the cases where the probe 01 has different impedance, this may be applied to a difference in the shape of the probe 01 connected to an ultrasonic diagnostic apparatus, for example, in a linear probe, a convex probe, or a sector probe.

The shape of the probe 01 differs with a region of an object to be measured, and the frequency band of the probe 01 is also different. For example, the linear probe is often applied to fine measurement objects, such as superficial parts on the surface of the human body, muscle fibers, and mammary gland, that is, to regions for which the spatial resolution is particularly required. For this reason, the driving frequency of the linear probe is higher than that of the convex probe and the sector probe in many cases.

In addition, even in the same probe, the effect is obtained by unique duty ratio setting according to the driving frequency. In general, since the impedance of a probe changes with frequency, it is necessary to change the setting of the probe according to the driving frequency even in the same probe.

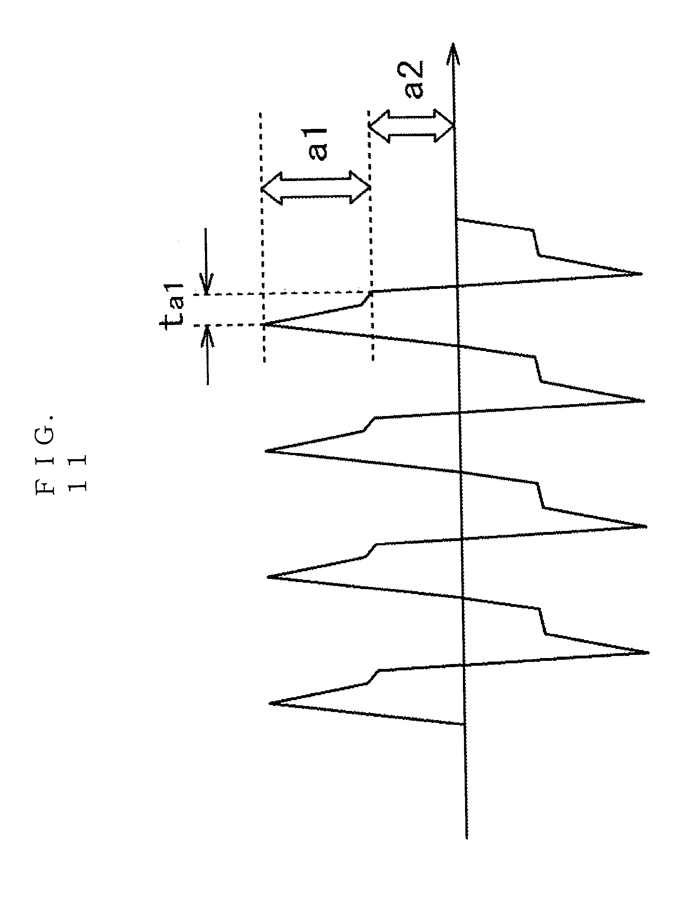

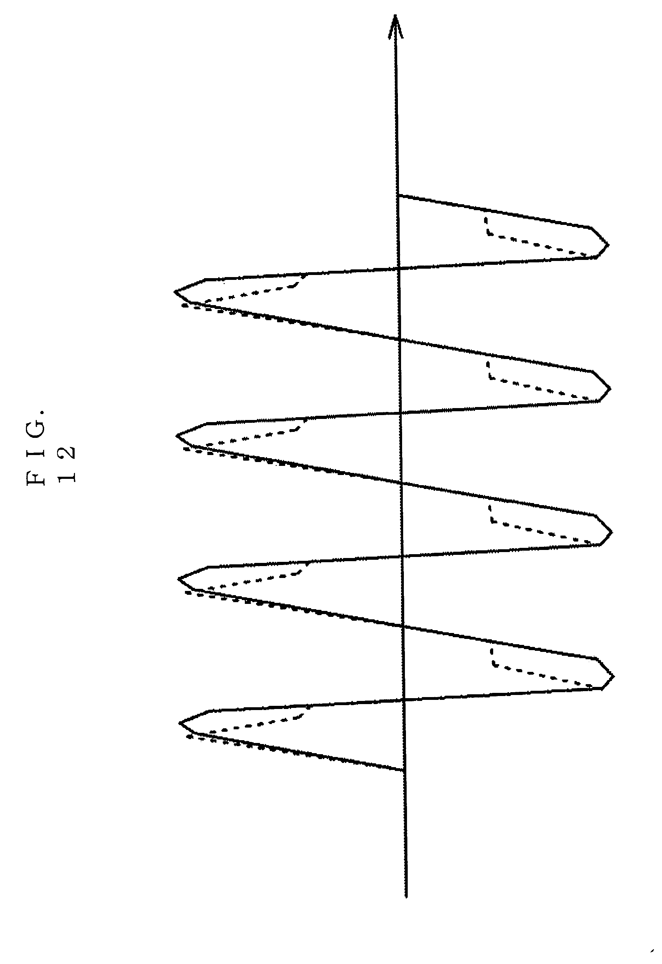

Fig. 11 shows an example of the transmission circuit output waveform when a probe is connected. This is a waveform with an output signal of four waves. As shown in Fig. 1, there are various loads between the signal transmission circuit 03 and the probe 01. The transmission and reception separation circuit 04, a change-over switch 15, the cable 06, and the like may be mentioned. They have a resistive component, a capacitive component, and a reactance component, and the transducer 05 in the probe also has a capacitive component. Therefore, when seen from the signal transmission circuit 03, this is equal to connection of loads with various time constants. For this reason, as shown in Fig. 11, an output signal may have a waveform with a time constant appearing in each place. Originally, a waveform of the solid line shown in Fig. 12 is preferable. An a1 portion shown in Fig. 11 is an influenced portion of a time constant resulting from the influence of various loads connected to a signal transmission circuit, a power supply, and the like, and an a2 portion is a portion where an original waveform output is obtained. Accordingly, time ta1 of the a1 portion is determined by the frequency of a driving signal and the load value at that time. Furthermore, a ratio of the a1 and a2 portions is determined by the output amplitude.

In addition, each of a non-inverted component and an inverted component with the output amplitude of this waveform is created by a separate circuit or the current path as shown in Fig. 10, for example. Accordingly, the time constant of the a1 portion appearing in the non-inverted component and the inverted component or the ratio of the a1 and a2 portions is influenced by the impedance of the signal path in which each amplitude is created. Therefore, they are not necessarily the same. For this reason, by generating a pulse signal obtained by changing the duty ratio in the transmission signal generator 14 as shown in Figs. 2 and 3 and performing control such that a step difference by the a1 and a2 portions shown in Fig. 11 is eliminated, for example, it becomes possible to suppress harmonic components by the addition waveform of the non-inverted component and the inverted component as a result.

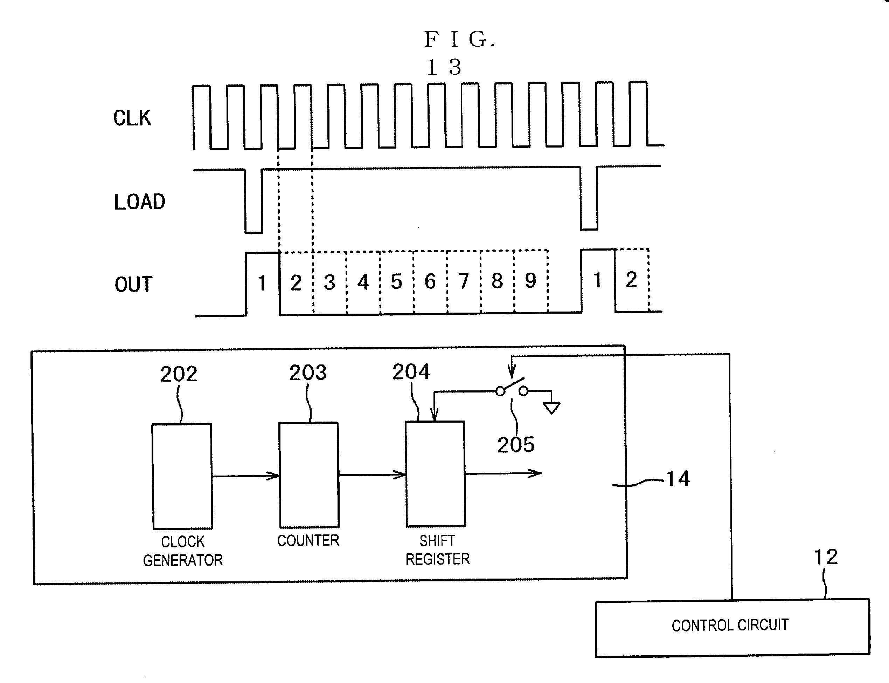

Moreover, in Fig. 10, the duty ratio of timing signals which are generated by the transmission signal generator 14 and are input to the FETs 200-1 and 200-2 is changed as shown in Fig. 13, for example.

In Fig. 13, the transmission signal generator 14 is configured to include a clock generator 202, a counter 203, and a shift register 204. The counter 203 is a timing generator circuit. In Fig. 13, the counter 203 outputs one LOAD signal in ten clocks, and the shift register 204 creates a duty ratio. The setting of the duty ratio may be changed by a switch 205, for example. The duty ratio is 0 when all switches 205 are ON, and an output of 100% is obtained when all switches 205 are OFF. In addition, the switch 205 is controlled by the control circuit 12. The control circuit 12 sets the optimum value according to the type of a connected probe, the frequency and amplitude of a transmission signal. As an example of the configuration for this, it is preferable to store in a memory a table showing the relationship between the duty ratio and the type of a connected probe, the amplitude of a transmission signal, and the frequency of a transmission signal, that is, the operating conditions of the change-over switch 15, so that the duty ratio corresponding to the type of a connected probe, the amplitude of a transmission signal, and the frequency of a transmission signal is set from the table.

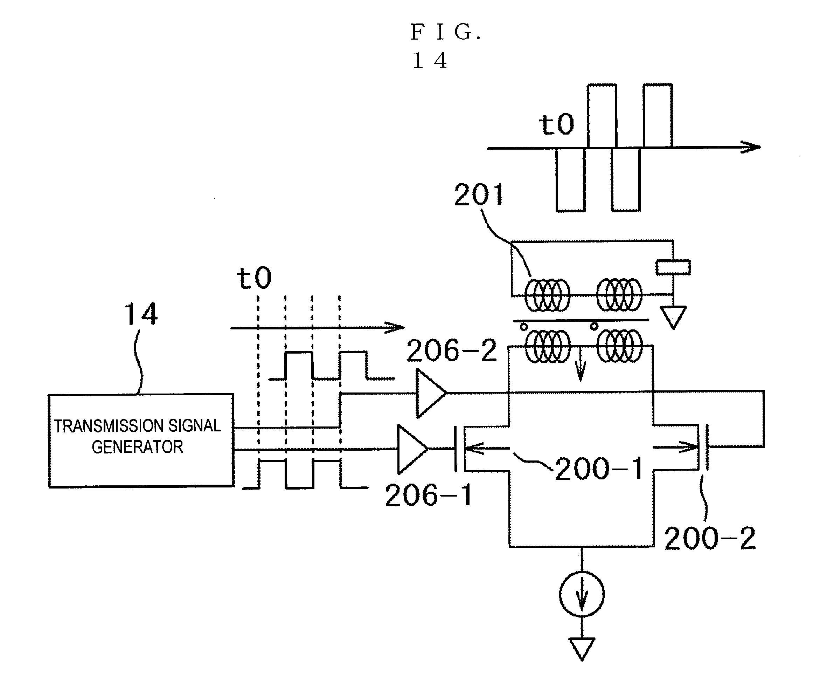

Fig. 14 is a circuit obtained by inserting buffer circuits 206-1 and 206-2 for current amplification in the connection path from the transmission signal generator 14 to the FETs 200-1 and 200-2 in the circuit shown in Fig. 10. Although the buffer circuits are used to assist the driving ability of FETs, there is generally a difference in the signal level switching time.

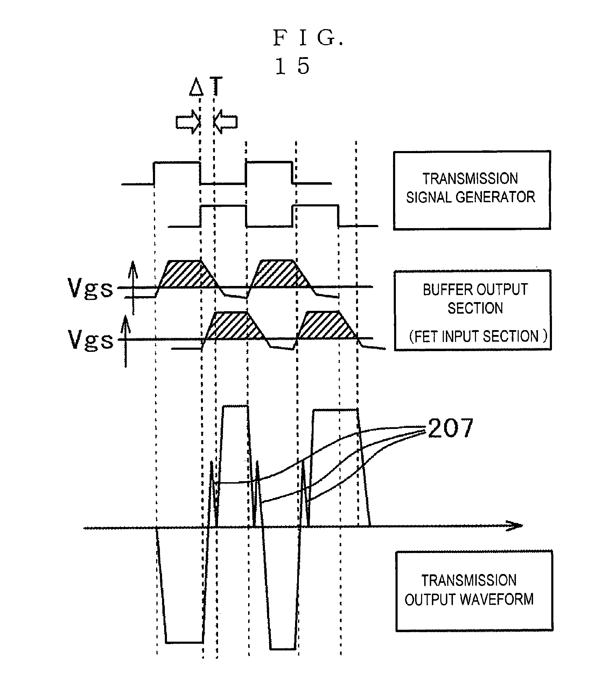

Fig. 15 shows a timing chart from a transmission signal generator to an output section of a signal transmission circuit. For the sake of convenience, a signal output from the transmission signal generator 14 is set as an ideal rectangular wave with no difference in signal level switching time. The following explanation will be given without taking variations due to components into consideration, but the present invention is not limited to this in practice.

In the buffer output section, for an ideal input signal, a time difference ΔT occurs in the signal level switching time (for example, rising time from the low signal level state to the high signal level state and falling time from the high signal level state to the low signal level state) due to the characteristics on the circuit. Here, the rising time and the falling time are defined as time required from Vgs, which is a threshold voltage in driving of the FET, to the maximum value of a rectangular signal.

In the buffer output section shown in Fig. 15, times for which the FETs 200-1 and 200-2 are turned on so that a current flows are shaded. Due to the time difference between rising and falling described above, a portion in which the times, for which the FETs 200-1 and 200-2 are turned on, overlap each other is present. Ideally, for example, when the FET 200-1 is responsible for a non-inverted component of the output signal of the signal transmission circuit, only this FET is turned on for the non-inverted component. On the other hand, when the FET 200-2 is responsible for an inverted component, only this FET is turned on for the inverted component. Accordingly, a current flows through the FETs 200-1 and 200-2 alternately.

However, since there is a time zone for which the FETs 200-1 and 200-2 are turned on at the same time temporarily due to the time difference between rising and falling, current flows through both the FETs 200-1 and 200-2. In this case, spike noise 207 which is a spike-like signal appears in the output signal of the signal transmission circuit. Since this signal includes many harmonic components, it appears as an unnecessary response in a diagnostic image on which harmonic imaging is performed. This causes a reduction in the S/N ratio.

In order to solve this, it is necessary to prevent the FETs 200-1 and 200-2 from being turned on simultaneously. Accordingly, it is preferable to generate a transmission signal with ON time, in which rising time and falling time in the entire transmission circuit system depending on electronic components used are considered, by the transmission signal generator 14 and to supply the transmission signal to the signal transmission circuit 03.

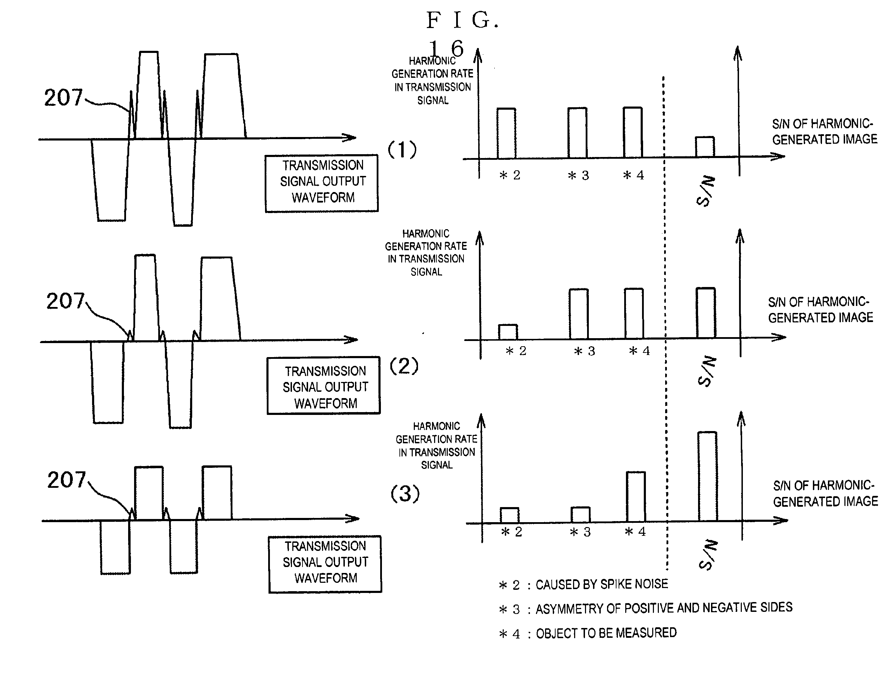

In Fig. 16, duty ratio setting when the above-described signal transmission circuit connection load is taken into consideration, the effect when rising time and falling time of a circuit system are corrected, and each contribution ratio are relatively expressed.

(1) shows a case where there is no correction. A harmonic caused by the asymmetry of a non-inverted component and an inverted component of a transmission output waveform and a harmonic component based on the spike noise generated by mismatch of the rising time and the falling time of the transmission circuit system are present together. Since it is difficult to distinguish these harmonic components from harmonic components from a measurement object to be actually imaged, an image with many noise components is generated.

(2) shows a case where a transmission signal in which the rising time and the falling time of the transmission circuit system do not match each other is supplied from the transmission signal generator 14. Although the harmonic component caused by the asymmetry of the non-inverted component and the inverted component of the transmission output waveform seldom changes, an improvement in the S/N ratio of a diagnostic image can be seen since the spike noise is suppressed.

In (3), the mismatch of the rising time and the falling time of the transmission circuit system is considered, and the duty ratio is set in consideration of the signal transmission circuit connection load. Accordingly, since the spike noise is reduced, a high-frequency component of an addition signal caused by the asymmetry of a non-inverted component and an inverted component can be reduced. As a result, the S/N ratio of a diagnostic image can be greatly improved.

According to the above configuration, for example, when the amplitude of an input transmission signal is equal to or larger than a fixed value, an output period of a signal with a value within the half period of the transmission signal can be set. When the amplitude of the transmission signal is smaller than the fixed value, it is possible to set no value within the half period of the transmission signal (ground level). Since an output signal of the signal transmission circuit does not depend on the duty ratio of an input signal of the signal transmission circuit, it is possible to reduce a harmonic component caused by changes in the signal level of an input signal to the signal transmission circuit.

In addition, the generation of harmonic components of a non-inverted component and an inverted component in an addition signal of the non-inverted component and the inverted component can be reduced by changing the duty ratio of the input signal to the signal transmission section. Since this duty ratio has an optimal value for at least one of a connected probe, the transmission frequency, amplitude, and a wave number, it is desirable to set this in the apparatus in advance. For example, it is preferable to store a table showing the relationship between the optimal duty ratio and the type of a connected probe, a transmission frequency, and transmission amplitude in a memory and read the duty ratio from the table and to set the duty ratio of the transmission signal.

The signal transmission circuit can contribute to reduction of high-frequency noise in the amplitude of a non-inverted component and the amplitude of an inverted component if the change-over switch 15 is provided in each of means for forming the non-inversion-side amplitude of the transmission signal and means for forming the inversion-side amplitude.

### Industrial Applicability

The present invention can be used for harmonic imaging, such as tissue harmonic imaging or contrast imaging, so that harmonic components caused by signal level changes in the transmission signal can be reduced.

The present invention is still more effective in nonlinear imaging using pulse inversion.

### Reference Signs List

- 01:: probe
- 02:: transmission signal phasing circuit
- 03:: signal transmission circuit
- 04:: transmission and reception separation circuit
- 05:: transducer
- 06:: probe cable
- 07:: received signal amplifier circuit
- 08:: received signal phasing processing circuit
- 09:: signal processing circuit
- 10:: image processing circuit
- 11:: display monitor
- 12:: control circuit
- 13:: power supply
- 14:: transmission signal generator
- 15:: change-over switch
- 100:: ultrasonic diagnostic apparatus
- 102, 103:: switching circuit
- 200-1, 2:: FET
- 201:: transformer
- 202:: clock generator
- 203:: counter
- 204:: shift register
- 205:: switch
- 206-1, 2:: buffer circuit
- 207:: spike noise

## Claims

1. An ultrasonic diagnostic apparatus comprising:
a signal transmission section which supplies a driving pulse to a transducer in order to transmit an ultrasonic transmission signal to an object and transmits to the object a non-inverted component and an inverted component of an ultrasonic waveform; and
a signal processing section which removes a fundamental wave component by adding received signals from the object,
wherein the signal transmission section includes a switching section which changes an output period within a half period of the transmission signal based on at least one of a load connected to the signal transmission section and amplitude, a frequency, and a wave number output from the signal transmission section.

2. The ultrasonic diagnostic apparatus according to claim 1,
wherein the signal transmission section is configured to be able to change an interval between the non-inverted component and the inverted component of the transmission signal.

3. The ultrasonic diagnostic apparatus according to claim 2,
wherein a field effect transistor connected to a ground is provided in an output end of the signal transmission section.

4. The ultrasonic diagnostic apparatus according to claim 1,
wherein the switching section of the signal transmission section is provided in each of means configured to form non-inversion-side amplitude of the transmission signal and means configured to form inversion-side amplitude.

5. The ultrasonic diagnostic apparatus according to claim 1,
wherein the signal transmission section further includes a memory in which operating conditions of the switching section are stored.

6. The ultrasonic diagnostic apparatus according to claim 4,
wherein a circuit which outputs the non-inversion-side amplitude of the transmission waveform and a circuit which outputs the inversion-side amplitude of the transmission waveform, which are included in the signal transmission section, are formed by components with the same characteristics.

7. The ultrasonic diagnostic apparatus according to claim 6,
wherein the signal transmission section has a circuit which amplifies an input signal using only one power supply on the non-inversion side or the inversion side, and each output section is commonly used through an inverting section and a non-inverting section of a transformer.

8. The ultrasonic diagnostic apparatus according to claim 6,
wherein a switching speed of a signal level of an input waveform of a circuit that supplies a driving pulse to a circuit, which outputs non-inversion-side amplitude of the transmission waveform, and a circuit, which outputs inversion-side amplitude of the transmission waveform, is changeable.
